(19) Europäisches Patentamt
European Patent Office
Office européen des brevets

(11) Veröffentlichungsnummer: **0 017 976**
**B1**

(12) **EUROPÄISCHE PATENTSCHRIFT**

(45) Veröffentlichungstag der Patentschrift: **22.06.83**

(21) Anmeldenummer: **80102042.1**

(22) Anmeldetag: **16.04.80**

(51) Int. Cl.³: **C 07 D 233/88,**
C 07 D 233/72,
C 07 C 127/19,
C 07 C 125/067,
A 61 K 31/415

# (54) Verfahren zur Herstellung von Imidazolidinderivaten.

(30) Priorität: **24.04.79 CH 3837/79**

(43) Veröffentlichungstag der Anmeldung:
**29.10.80 Patentblatt 80/22**

(45) Bekanntmachung des Hinweises auf die Patenterteilung:
**22.06.83 Patentblatt 83/25**

(84) Benannte Vertragsstaaten:
**AT BE CH DE FR GB IT LI LU NL SE**

(56) Entgegenhaltungen:
EP - A - 0 001 813
DE - A - 1 958 183
DE - A - 2 649 925
FR - A - 2 407 205
GB - A - 997 037
US - A - 3 340 070

(73) Patentinhaber: **F. HOFFMANN-LA ROCHE & CO. Aktiengesellschaft**
**CH-4002 Basel (CH)**

(72) Erfinder: **Link, Helmut, Dr.**
**Dammerkirchstrasse 70**
**CH-4056 Basel (CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

(56) Entgegenhaltungen:
JOURNAL OF THE AMERICAN CHEMICAL SOCIETY, Band 67, 15. November 1945, Seiten 1996—1998 Washington D.C., U.S.A.
R. A. JACOBSON: "N-Substituted alfa-aminoisobutyronitriles from acetone cyanohydrin"
Houben-Weyl, Methoden der organischen Chemie, Bd. VIII (1952), Seiten 161—162

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 017 976 B1

# Verfahren zur Herstellung von Imidazolidinderivaten

Die Erfindung betrifft ein neues Verfahren zur Herstellung von Imidazolidin-derivaten der allgemeinen Formel

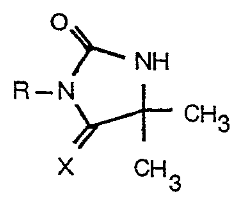

I

worin X Imino oder Sauerstoff und R eine der Gruppen

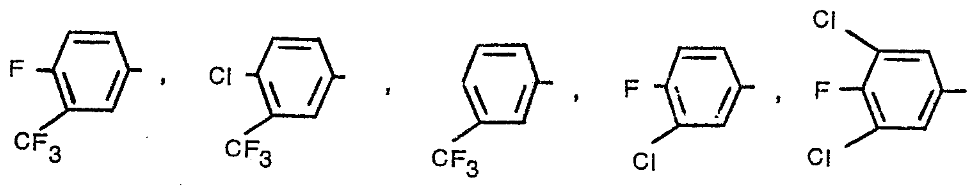

darstellt.

Aus der britischen Patentschrift No. 997 037 ist es bekannt, Hydantoinderivate herzustellen, durch Umsetzung eines $\alpha$-Amino-isobutyronitriles mit Isocyanaten und anschliessender Cyclisierung der zunächst entstandenen Harnstoffverbindung. Die obigen Verbindungen der Formel I sind als solche Gegenstand der älteren europäischen Patentanmeldung No. 78.101244.8 (EP—A—0001813).

Die nach dem erfindungsgemässen Verfahren erhältlichen Produkte der Formel I besitzen wertvolle therapeutische Eigenschaften und können dementsprechend als Arzneimittel Verwendung finden. Beispielsweise zeichnen sie sich durch antiandrogene Wirkung aus und können demgemäss z.B. zur Behandlung von Krankheiten, die mit einer erhöhten androgenen Aktivität in Verbindung stehen, wie z.B. Akne, Seborrhoe, Hersutimus und Prostata-adenom verwendet werden. Besonders bevorzugte, antiandrogen wirksame Verbindungen der Formel I sind diejenigen, in denen X Sauerstoff darstellt (vgl. die nachstehende Formel Ib); wie z.B. das besonders bevorzugte 3-(3-Chlor-4-fluor-phenyl)-5,5-dimethyl-hydantoin. Diejenigen Imidazolidinderivate der Formel I, in denen R z.B. 3-Trifluormethyl-4-fluor-phenyl, 3-Trifluormethyl-4-chlor-phenyl, 3-Trifluormethyl-phenyl oder 3-Chlor-4-fluor-phenyl bedeutet, sind schistosomicid wirksam und können somit zur Therapie und Verhütung der Bilharziose eingesetzt werden. Ganz besonders bevorzugt wegen ihrer starken schistosomiciden Wirkung sind 3-(3-Trifluormethyl-4-fluor-phenyl)-5,5-dimethyl-hydantoin und 1-(3-Trifluormethyl-4-fluor-phenyl)-5-imino-4,4-dimethyl-2-imidazolidinon.

Die antiandrogene Wirkung wurde durch Bestimmung der Gewichtsabnahme der ventralen Prostata und der Samenblase von männlichen sterilisierten Ratten ermittelt.

Die schistosomicide Wirkung wurde ermittelt durch Vergleich des prozentualen Anteils lebender und toter Wurmpaare in den Gefässen der Leber sowohl bei mit Cercarien von Schistosoma mansoni infizierten, benandelten Mäusen als auch bei infizierten aber unbehandelten Kontrolltieren. Die Bestimmung der $VD_{50}$ (Vermicide Dosis 50%: Dosis, die 50% der Wurmpaare abtötet) erfolgte nach der Probitmethode.

Die erfindungsgemäss erhältlichen Produkte können als Heilmittel, z.B. in Form pharmazeutischer Präparate Verwendung finden, welche sie in Mischung mit einem für die enterale oder parenterale Applikation geeigneten pharmazeutischen organischen oder anorganischen Trägermaterial, wie Gelatine, Milchzucker, Stärke, Gummi arabicum, Magnesiumstearat, Talcum, pflanzliche Oele, Polyalkylenglykole, Vaseline u.dgl. mehr enthalten. Die Präparate können in fester Form, z.B. als Tabletten, Dragées, oder in flüssiger Form, z.B. als Lösungen, Suspensionen oder Emulsionen, vorliegen. Sie können Hilfsstoffe, wie Konservierungs-, Stabilisierungs-, Netz- oder Emulgiermittel, Salze zur Veränderung des osmotischen Druckes oder Puffer enthalten. Auch andere therapeutisch wirksame Stoffe können beigemischt sein.

Zweckmässige pharmazeutische Dosierungsformen enthalten etwa 10—500 mg einer Verbindung der Formel I.

Die Dosierung wird entsprechend den individuellen Erfordernissen gewählt. Zum Beispiel können diese Verbindungen in Dosierungen von etwa 0,1 mg/kg bis etwa 50 mg/kg pro Tag p.o., verabreicht werden.

Als antiandrogene Mittel verwendbare Dosierungsformen enthalten zweckmässig etwa 10—500 mg, vorzugsweise etwa 100 mg Wirkstoff. Die Dosierung ist beispielsweise etwa 0,1 mg/kg

bis etwa 10 mg/kg pro Tag p.o., vorzugsweise etwa 1 mg/kg pro Tag p.o. Zweckmässigerweise wird diese Dosis in Anpassung an den Zustand des Patienten etwa 3—8 Monate täglich verabreicht.

Als schistosomicide Mittel verwendbare Dosierungsformen enthalten zweckmässig etwa 100—500 mg, vorzugsweise etwa 250 mg Wirkstoff. Die Dosierung ist beispielsweise etwa 5 mg/kg bis etwa 50 mg/kg pro Tag p.o., vorzugsweise 25 mg/kg p.o. pro Tag. Diese Menge kann in einer einzelnen Dosierung oder in mehreren unterteilten Dosierungen verabreicht werden, je nach Bedürfnis des Patienten und Vorschrift des Fachmannes. Zweckmässigerweise wird diese Dosis in Anpassung an den Zustand des Patienten an einem oder an mehreren aufeinanderfolgenden Tagen verabreicht.

Das erfindungsgemässe Verfahren zur Herstellung der Verbindungen der Formel I ist dadurch gekennzeichnet, dass man eine Verbindung der allgemeinen Formel

$$R—NH—R^4 \qquad II$$

mit einer Verbindung der allgemeinen Formel

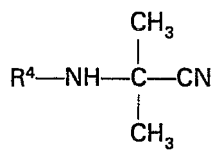

III

wobei in den Formeln II und III das eine $R^4$ Wasserstoff und das andere $R^4$ eine $COOR^5$ Gruppe bedeutet, worin $R^5$ gegebenenfalls substituiertes Phenyl darstellt und R dasselbe wie oben bedeutet, zu einer Verbindung der allgemeinen Formel

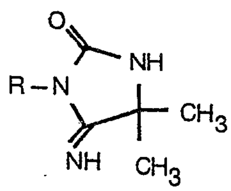

Ia

worin R dasselbe wie oben bedeutet,
umsetzt und erwünschtenfalls die letztere Verbindung zu einer Hydantoinverbindung der allgemeinen Formel

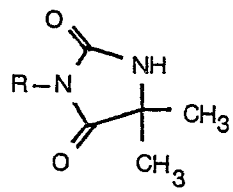

Ib

worin R dasselbe wie oben bedeutet,
hydrolysiert.

Zur Gewinnung der Verbindungen der Formel Ia kann man nach einer ersten Variante eine Verbindung der Formel II mit $R^4 = COOR^5$ also eine Verbindung der Formel

$$R—NH—COOR^5 \qquad IIa$$

worin R und $R^5$ dasselbe wie oben bedeuten,
mit einer Verbindung der Formel III mit $R^4 = H$, also mit der Verbindung

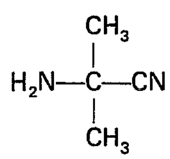

IIIa

(α-Amino-isobutyro-nitril)
oder nach einer zweiten Variante eine Verbindung II mit $R^4 = H$, also eine Verbindung der Formel

R—$NH_2$ IIb

worin R dasselbe wie oben bedeutet,
mit einer Verbindung II mit $R^4 = COOR^5$, also eine Verbindung der Formel

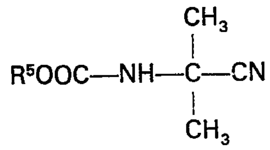 IIIb

worin $R^5$ dasselbe wie oben bedeutet,
umsetzen.

Die Umsetzung nach den beiden Varianten erfolgt zweckmässig in Lösung, wobei als Lösungsmittel z.B. folgende in Betracht kommen: Alkohole, wie niedere Alkanole, vorzugsweise sekundäre, wie insbesondere Isopropanol; Aether, wie beispielsweise Tetrahydrofuran; Säureamide, wie Dimethylformamid. Die Umsetzung erfolgt ferner zweckmässig in Gegenwart einer Base, wie Natriumbicarbonat oder Triäthylamin, wobei zweckmässig äquivalente Mengen an Base wie auch an Reaktionspartnern (IIa und IIIa bzw. IIb und IIIb) eingesetzt werden. Die Reaktion wird zweckmässig bei ungefähr Raumtemperatur durchgeführt. Doch kommen auch tiefere oder höhere Temperaturen, z.B. bis zu den Siedetemperaturen der Lösungen, in Frage.

Die gegebenenfalls erwünschte Umwandlung einer Verbindung Ia in eine Hydantoinverbindung Ib kann mittels Hydrolyse, zweckmässig saurer Hydrolyse, z.B. mit Mineralsäuren, wie Salzsäure, bei Temperaturen zwischen Raumtemperatur und der Siedetemperatur der Reaktionslösung durchgeführt werden.

Die als Ausgangssubstanzen eingesetzten Verbindungen IIa, IIb, IIIa und IIIb können, soweit sie nicht bekannt sind, nach an sich bekannten Methoden gewonnen werden. So können die Verbindungen IIa durch Umsetzung eines Amins R—$NH_2$ (IIb) mit einem Chlorameisenphenylester Cl—COO—$R^5$ erhalten werden und die Verbindungen IIIb durch Umsetzung eines solchen Esters (Cl—COO—$R^5$) mit $\alpha$-Amino-isobutyronitril (IIIa).

Bei der erfindungsgemässen Umsetzung einer Verbindung II mit einer Verbindung III können in bestimmten Fällen, wie z.B. bei Verwendung von p-Nitrophenyl als $R^5$-Rest, noch nicht cyclische Verbindungen der Formel

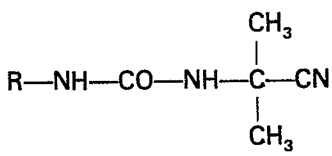 IV

worin R dasselbe wie oben bedeutet,
auftreten und erwünschtenfalls isoliert werden. Sie cyclisieren unter den zu ihrer Bildung führenden Reaktionsbedingungen oder in Form einer Schmelze leicht zu den Verbindungen Ia.

In den nachfolgenden Beispielen sind die Temperaturen in Celsiusgraden angegeben.

## Beispiel 1

a) Zu einer Lösung von 4,4 g (14,7 mMol) 3-Trifluormethyl-4-fluor-phenylcarbamidsäure-phenylester in 20 ml Isopropanol werden 1,28 g (15,2 mMol) $\alpha$-Aminoisobutyronitril und 1,28 g (15,2 mMol) Natriumbicarbonat gegeben. Nach 18-stündigem Rühren wird die Suspension mit 100 ml Aether verdünnt, filtriert und eingedampft. Das Produkt wird in 200 ml Aether gelöst, die Lösung mit gesättigter Natriumbicarbonatlösung und Wasser gewaschen und mit 200 ml 0,1n Salzsäure extrahiert. Die salzsaure Lösung wird unter Eiskühlung mit Natriumbicarbonat alkalisch (pH 8) gestellt und mit Aether extrahiert. Nach Trocknen über Natriumsulfat und Eindampfen der ätherischen Lösung wird das Produkt aus Aether/Petroläther kristallisiert. Man erhält nach Trocknen bei 40° unter stark vermindertem Druck 1-(3-Trifluormethyl-4-fluor-phenyl)-5-imino-4,4-dimethyl-2-imidazolidinon vom Smp. 134—135°.

b) Das erhaltene 5-Imino-2-imidazolidinon kann wie folgt zum entsprechenden Hydantoin hydrolysiert werden:

Eine Suspension von 0,72 g (2,5 mMol) 1-(3-Trifluormethyl-4-fluor-phenyl)-5-imino-4,4-dimethyl-2-imidazolidinon in 10 ml 3n Salzsäure wird 4 Stunden bei 60° gerührt. Dabei löst sich das Edukt auf und das Produkt fällt allmählich aus. Nach dem Abkühlen wird es mit Methylenchlorid extrahiert. Die organische Phase wird über Natriumsulfat getrocknet und konzentriert. Durch Zugabe von Petroläther wird das Produkt ausgefällt und anschliessend aus Aether/Petroläther umkristallisiert. Man erhält nach Trocknen bei 50° unter stark vermindertem Druck 3-(3-Trifluormethyl-4-fluorphenyl)-5,5-dimethyl-hydantoin vom Smp. 111—112°.

c) Der als Ausgangsstoff verwendete Carbamidsäureester kann wie folgt erhalten werden:

Zu einer Lösung von 35,8 g (0,2 Mol) 5-Amino-2-fluorbenzotrifluorid in 300 ml abs. Tetrahydrofuran werden 16,8 g (0,2 Mol) Natriumbicarbonat gegeben. Unter Rühren werden 31,3 g (0,2 Mol) Chlorameisensäurephenylester gelöst in 100 ml abs. Tetrahydrofuran zugetropft, wobei die Temperatur zwischen 20 und 30° gehalten wird. Nach einer halben Stunde werden 350 ml Methylenchlorid zugefügt. Die Lösung wird mit Wasser gewaschen, über Natriumsulfat getrocknet und eingeengt. Das Prokukt wird aus Petroläther kristallisiert. Man erhält nach Trocknen bei 35° unter stark vermindertem Druck 3-Trifluoromethyl-4-fluor-phenyl-carbamidsäure-phenylester vom Smp. 115—116°.

## Beispiel 2

a) Zu 15 ml Isopropanol werden 2,65 g (10 mMol) 3-Chlor-4-fluor-phenylcarbamidsäure-phenylester, 840 mg (10 mMol) Natriumbicarbonat und 840 mg (10 mMol) $\alpha$-Aminoisobutyronitril gegeben. Nach 36-stündigem Rühren bei Raumtemperatur wird eingeengt, der Rückstand in 50 ml Aether aufgenommen, zuerst mit 20 ml gesättigter Natriumbicarbonatlösung und dann mit Wasser gewaschen. Die ätherische Lösung wird mit 130 ml 0,1n Salzsäure und Wasser extrahiert. Die wässrige Lösung wird unter Eiskühlung mit Natriumbicarbonat alkalisch (pH 8) gestellt und mit Methylenchlorid extrahiert. Nach Trocknen über Natriumsulfat und Eindampfen des Extraktes wird das verbleibende Produkt aus Aether/Petroläther kristallisiert. Man erhält nach Trocknen bei 50° unter stark vermindertem Druck 1-(3-Chlor-4-fluorphenyl)-5-imino-4,4-dimethyl-2-imidazolidinon vom Smp. 135—136°.

b) Das erhaltene 5-Imino-2-imidazolidinon kann in Analogie zu Beispiel 1b mit 1n HCl unter Rühren bei Raumtemperatur während 3 Tagen zum entsprechenden Hydantoin [3-(3-Chlor-4-fluor-phenyl)-5,5-dimethylhydantoin, Smp. 165—166°) hydrolysiert werden.

c) Der als Ausgangsstoff verwendete Carbamidsäureester kann wie folgt erhalten werden:

Eine Lösung von 7,25 g (50 mMol) 3-Chlor-4-fluoranilin in 90 ml abs. Tetrahydrofuran wird mit 4,2 g (50 mMol) Natriumbicarbonat und 7,8 g (50 mMol) Chlorameisensäurephenylester versetzt und 1/2 Stunde bei Raumtemperatur gerührt. Anschliessend wird die Lösung nach Zugabe von 300 ml Methylenchlorid und 80 ml Wasser geschüttelt und die organische Phase abgetrennt. Nach Trocknen über Natriumsulfat wird die Lösung eingedampft und das verbleibende Produkte zweimal aus Aether/Petroläther kristallisiert. Man erhält nach Trocknen bei 40° unter stark Vermindertem Druck 3-Chlor-4-fluor-phenylcarbamidsäure-phenylester vom Smp. 111—112°.

## Beispiel 3

a) 3,44 g (10 mMol) 3-Trifluormethyl-4-fluor-phenylcarbamidsäure-4'-nitrophenylester, 50 ml Isopropanol und 0,84 g (10 mMol) $\alpha$-Aminoisobutyronitril werden zusammengegeben. Nach 10-minütigem Rühren bei Raumtemperatur wird die entstandene gelbe Lösung eingeengt und das verbleibende Produkt mit 200 ml Aether Aufgenommen. Die Lösung wird mit 160 ml gesättigter Natriumbicarbonatlösung gewaschen, über Natriumsulfat getrocknet und konzentriert. Dabei kristallisiert das Produkt aus. Nach Trocknen bei 50° unter stark vermindertem Druck erhält man N-(2-Cyano-2-methyläthyl)-N'-[4-fluor-3-(trifluormethyl)phenyl]-harnstoff vom Smp. 132—133°. Vgl. Formel IV

Ein Lösung von 145 mg (0,5 mMol) des so erhaltenen N-(2-Cyano-2-methyläthyl)-N'-[4-fluor-3-(trifluormethyl)phenyl]-harnstoff wird 8 Stunden gerührt und eingedampft. Das Produkt wird aus Aether/n-Pentan kristallisiert. Man erhält 1-(3-Trifluormethyl-4-fluorphenyl)-5-imino-4,4-dimethyl-2-imidazolidinon vom Smp. 132—135°.

b) Der als Ausgangsstoff eingesetzte Carbamidsäureester kann wie folgt erhalten werden:

Zu einer Lösung von 1,79 g (10 mMol) 5-Amino-2-fluorbenzotrifluorid in 30 ml abs. Tetrahydrofuran werden 2,01 g (10 mMol) Chlorameisensäure-4-nitrophenylester und 0,84 g (10 mMol) Natriumbicarbonat gegeben. Nach 1/2 Stunde Rühren bei Raumtemperatur wird das Gemisch filtriert, das Nutschgut mit Aether gewaschen und Filtrat und Waschlösung zusammen eingeengt. Das Produkt wird zweimal aus Aether/Petroläther kristallisiert. Nach Trocknen bei 50° unter stark vermindertem Druck erhält man 3-Trifluormethyl-4-fluor-phenylcarbamidsäure-4'-nitrophenylester vom Smp. 129—133°.

## Beispiel 4

a) Eine Lösung von 179mg (1 mMol) 5-Amino-2-fluorbenzotrifluorid in 3 ml Isopropanol wird mit 84 mg (1 mMol) Natriumbicarbonat und 204 mg (1 mMol) (1-Cyan-1-methyläthyl)carbamidsäurephenylester versetzt und 16 Stunden bei Raumtemperatur gerührt. Nach Zugabe von 20 ml Aether wird die Lösung mit gesättigter Natriumbicarbonatlösung und Wasser gewaschen und mit 12 ml 0,1n Salzsäure extrahiert. Die salzsaure Lösung wird unter Eiskühlung mit Natriumbicarbonat wieder alkalisch gestellt (pH 8) und mit Methylenchlorid extrahiert. Nach Trocknen über Natriumsulfat und Eindampfen der Lösung wird das Produkt aus Aether/Petroläther kristallisiert. Man erhält 1-(3-Trifluormethyl-4-fluor-phenyl)-5-imino-4,4-dimethyl-2-imidazolidinon vom Smp. 134—135°.

b) Der als Ausgangsstoff eingesetzte Carbamidsäureester kann wie folgt erhalten werden:

In einer Lösung von 840 mg (10 mMol) $\alpha$-Aminoisobutyronitril in 10 ml abs. Tetrahydrofuran werden 840 mg (10 mMol) Natriumbicarbonat suspendiert. 1,56 g (10 mMol) Chlorameisensäurephenylester werden unter Rühren zuge-tropft, wobei die Temperatur zwischen 20 und 30° gehalten wird. Nach weiteren 6 Stunden wird filtriert, das Nutschgut mit Aether Ausgewaschen, Filtrat und

Waschlösung eingedampft und das Produkt aus Aceton/Petroläther kristallisiert. Man erhält nach Trocknen bei 50° unter stark vermindertem Druck (1-Cyan-1-methyl-äthyl)carbamidsäure-phenylester vom Smp. 116—118°.

Beispiel 5

In Analogie zu den obigen Beispielen 1—4 kann über das entsprechende 5-Imino-2-imidazolidinon die folgende Hydantoin-Verbindung erhalten werden:

3-(3-Trifluormethyl-phenyl)-5,5-dimethylhydantoin; Smp. 175—176°.

Beispiel 6

In einem 2,5 Liter-Sulfierkolben, der mit Rührer, Thermometer, Tropftrichter und Calciumchloridrohr versehen ist, wird eine Lösung von 187 g $\alpha,\alpha,\alpha$,4-Tetrafluor-m-tolylcarbamidsäurephenylester in 700 ml Tetrahydrofuran (absolutiert durch Chromatographie an $Al_2O_3$) vorgelegt. Unter Argonbegasung werden 52,6 g Natriumhydrogencarbonat und eine Lösung von 52,6 g $\alpha$-Aminoisobutyronitril in 50 ml Tetrahydrofuran (absolutiert durch Chromatographie an $Al_2O_3$) zugegeben. Nach 40-stündigem Rühren bei Raumtemperatur werden ca. 700 ml Aether zugefügt. Die ätherische Lösung wird nach 10 Min. Stehen vom Salz abgetrennt und mit ca. 300 ml Eis-Wasser versetzt. Nach intensivem Schütteln wird die wässrige Phase abgetrennt und mit 2 x 100 ml Aether gewaschen. Die vereinigten ätherischen Lösungen werden auf ca. —5°C gekühlt und mit ca. 600 ml Eis-Wasser und 700 ml eiskalter 1n HCl versetzt. Nach intensivem Schütteln wird die saure wässerige Lösung abgetrennt und die ätherische Lösung noch mit 3 x 100 ml eiskaltem Wasser gewaschen. Die vereinigten wässerigen Lösungen werden vorsichtig unter intensiver Kühlung (Innentemperatur ca. 3°C) und Rühren mit 65,6 g Natriumhydrogencarbonat schwach alkalisch gestellt und mit ca. 5 x 1200 ml Methylenchlorid extrahiert. Der Extrakt wird über Natriumsulfat getrocknet und unter vermindertem Druck bei 40° eingedampft. Der verbleibende schaumige Rückstand wird in ca. 100 ml Aether gelöst und bis zur Kristallisation mit Petroläther (tiefsiedend) versetzt. Man erhält nach 18-stündigem Trocknen bei 45° unter stark vermindertem Druck 148,8 g 1-(3-Trifluormethyl-4-fluor-phenyl)-5-imino-4,4-dimethyl-2-imidazolidinon (82% d. Th., Smp. 133—134°).

Die erhaltene kristalline, reine Substanz ist farblos.

Das erhaltene 5-Imino-2-imidazolidinon kann wie folgt zum entsprechenden Hydantoin hydrolysiert werden:

Eine Lösung von 5,78 g 1-(3-Trifluormethyl-4-fluor-phenyl)-5-imino-4,4-dimethyl-2-imidazolidinon in 30 ml 3n Salzsäure wird 4 1/2 Stunden bei 60° (Aussentemperatur) gehalten. Dabei fällt das Produkt kristallin aus. Es wird abfiltriert, mit deionisiertem Wasser gewaschen und in Aether gelöst. Die Lösung wird über Natriumsulfat getrocknet und konzentriert. Das Produkt wird durch Zugabe von n-Pentan kristallisiert. Nach 16-stündigem Trocknen bei 50° unter stark vermindertem Druck erhält man 5,7 g 3-(3-Trifluormethyl-4-fluorphenyl)-5,5-dimethyl-hydantoin (98% d. Th., Smp. 112—113°).

Die erhaltene kristalline, reine Substanz ist farbund geruchlos.

Der als Ausgangsstoff verwendete Carbamidsäureester kann wie folgt erhalten werden:

In einem 4,5 Liter-Sulfierkolben, der mit Rührer, Thermometer, Tropftrichter und Calciumchloridrohr ausgerüstet ist, wird eine Lösung von 107,5 g 5-Amino-2-fluorbenzotrifluorid in 800 ml Tetrahydrofuran (absolutiert durch Chromatographie an $Al_2O_3$) vorgelegt. Unter Rühren werden 50,4 g Natriumhydrogencarbonat zugegeben. Bei 22 bis 23°C Innentemperatur wird innert 5 bis 10 Min. eine Lösung von 94,0 g Chlorameisensäurephenylester in 200 ml Tetrahydrofuran (absolutiert durch Chromatographie an $Al_2O_3$) zugetropft und anschliessend 1/2 Stunde bei 23° weitergerührt. Es werden 600 ml Methylenchlorid zugefügt und das Gemisch zuerst mit 500 ml, dann mit 200 ml ionenfreiem Wasser gewaschen. Die wässrigen Lösungen werden mit 3 x 100 ml Methylenchlorid ausgezogen. Die vereinigten Methylenchlorid-Extrakte werden über Natriumsulfat getrocknet und eingeengt. Durch Zugabe von Petroläther (tiefsiedend) wird das Produkt kristallisiert. Man erhält nach 16-stündigem Trocknen bei 50° unter stark vermindertem Druck 175 g 3-Trifluormethyl-4-fluor-phenyl-carbamidsäure-phenylester (98% d. Th.; Smp. 115—116°).

Die erhaltene kristalline Substanz ist farb- und geruchlos.

**Patentansprüche**

1. Verfahren zur Herstellung von Imidazolidinderivaten der allgemeinen Formel

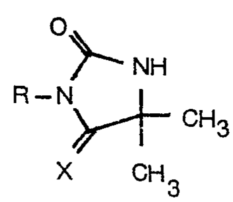

I

worin X Imino oder Sauerstoff und R eine der Gruppen

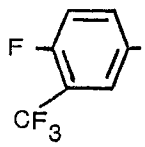 , 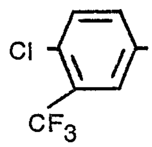 , 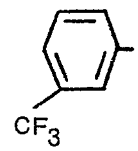 , 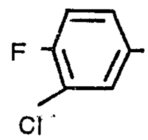 , 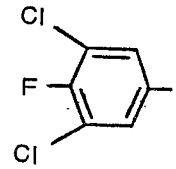

darstellt,
dadurch gekennzeichnet, dass man eine Verbindung der allgemeinen Formel

$$R—NH—R^4 \qquad II$$

mit einer Verbindung der allgemeinen Formel

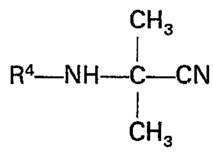

III

wobei in den Formeln II und III das eine $R^4$ Wasserstoff und das andere $R^4$ eine $COOR^5$ Gruppe bedeutet, worin $R^5$ gegebenenfalls substituiertes Phenyl darstellt und R dasselbe wie oben bedeutet, zu einer Verbindung der allgemeinen Formel

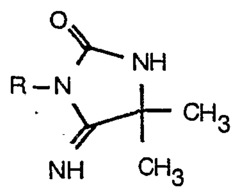

Ia

worin R dasselbe wie oben bedeutet,
umsetzt und erwünschtenfalls die letztere Verbindung zu einer Hydantoinverbindung der allgemeinen Formel

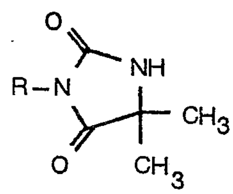

Ib

worin R dasselbe wie oben bedeutet,
hydrolysiert.

2. Verfahren nach Anspruch 1, dadurch gekennzeichnet, dass $R^5$ p-Nitrophenyl bedeutet.

**Revendications**

1. Procédé de préparation de dérivés de l'imidazolidine répondant à la formule générale:

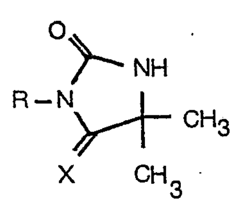

I

dans laquelle
X représente un groupe imino ou l'oxygène et
R représente l'un des groupes:

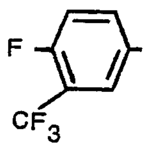, 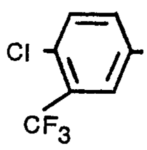, 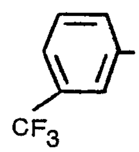, 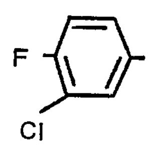, 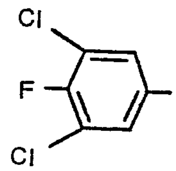.

caractérisé en ce que, en faisant réagir un composé de formule générale:

$$R{-}NH{-}R^4 \qquad II$$

avec un composé de formule générale:

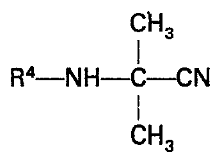

III

l'un des symboles $R^4$ des formules II et III représentant l'hydrogène et l'autre un groupe $COOR^5$ dans lequel $R^5$ est un groupe phényle éventuellement substitué, R ayant les significations indiquées ci-dessus,
on forme un composé de formule générale:

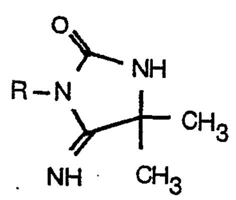

Ia

dans laquelle R a les significations indiquées ci-dessus, et si on le désire on convertit ce dernier composé, par hydrolyse, en un dérivé de l'hydantoïne répondant à la formule générale:

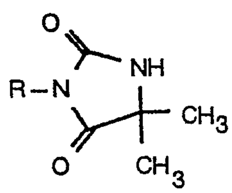

Ib

dans laquelle R a les significations indiquées ci-dessus.

2. Procédé selon la revendication 1, caractérisé en ce que $R^5$ représente le groupe p-nitrophényle.

## Claims

1. Process for the manufacture of imidazolidine derivatives of the general formula

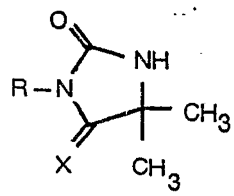

I

wherein X represents imino or oxygen and R represents one of the groups

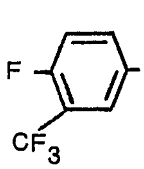 , 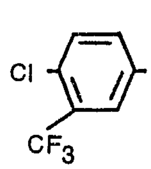 , 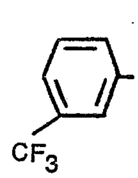 , 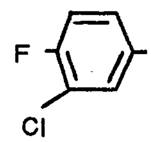 , 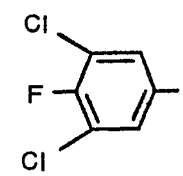

characterized by reacting a compound of the general formula

$$R—NH—R^4 \qquad II$$

with a compound of the general formula

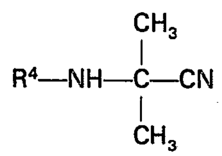

III

whereby in formulae II and III the one $R^4$ signifies hydrogen and the other $R^4$ signifies a $COOR^5$ group, wherein $R^5$ represents optionally substituted phenyl, and R signifies the same as above, to a compound of the general formula

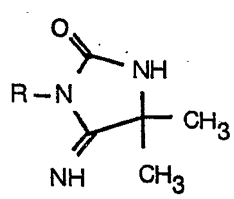

Ia

wherein R signifies the same as above, and, if desired, hydrolyzing the latter compound to a hydantoin compound of the general formula

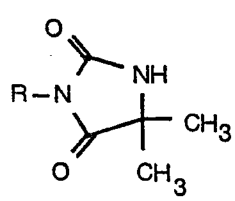

Ib

wherein R signifies the same as above,

2. Process according to claim 1, characterized in that $R^5$ signifies p-nitrophenyl.